# EUROPEAN PATENT SPECIFICATION

(11) **EP 3 926 073 B1**
(45) Date of publication and mention of the grant of the patent: **27.03.2024**
(21) Application number: 20788381.0
(22) Date of filing: 08.04.2020
(51) Int. Cl.: C23C 24/08, A61L 27/04, A61L 27/06, A61L 27/32

(54) **METHOD FOR FIXING HYDROXYAPATITE TO TITANIUM METAL SUBSTRATE, AND HYDROXYAPATITE-COATED METAL MATERIAL**
VERFAHREN ZUR FIXIERUNG VON HYDROXYAPATIT AUF EINEM TITANMETALLSUBSTRAT UND HYDROXYAPATITBESCHICHTETES METALLMATERIAL
PROCÉDÉ DE FIXATION D'HYDROXYAPATITE SUR UN SUBSTRAT MÉTALLIQUE À BASE DE TITANE, ET MATÉRIAU MÉTALLIQUE REVÊTU D'HYDROXYAPATITE

(30) Priority: 09.04.2019 JP 2019074248
(43) Date of publication of application: 22.12.2021
(73) Proprietor: Castem Co., Ltd., Fukuyama-city, Hiroshima 720-0004 (JP)
(72) Inventor: SHIRAISHI, Kohei, Higashi-Hiroshima City, Hiroshima 739-2116 (JP); SAGARA, Shusaku, Fukuyama-city, Hiroshima 720-0004 (JP); TODA, Takuo, Fukuyama-city, Hiroshima 720-0004 (JP)
(74) Representative: Gille Hrabal Partnerschaftsgesellschaft mbB Patentanwälte
(86) International application number: PCT/JP2020/015782
(87) International publication number: WO 2020/209278

(56) References cited:
- CN-A- 102 059 209
- JP-A- 2007 075 485
- JP-A- 2007 075 486
- KR-A- 20090 083 525
- US-A- 5 077 132
- US-A1- 2010 243 429
- US-A1- 2018 250 200
- HWANG, K.E et al.: "Interface characteristics changed by heat treatment of Ti materials with hydroxyapatite", Materials Science & Engineering C, vol. 23, no. 3, 2003, pages 401-405, XP55749902, ISSN: 0928-4931

## Description

### TECHNICAL FIELD

The present invention relates to a method for fixing hydroxyapatite (Ca₁₀(PO₄)₆(OH)₂) to a titanium-based metal substrate by a sintering method, and a hydroxyapatite-coated metal material. More specifically, it relates to a hydroxyapatite-coated metal material suitably used for medical materials such as artificial bones, artificial joints, implant, and the like, which becomes a foothold (scaffold) for osteoblasts to proliferate. In the present specification, hydroxyapatite may be sometimes abbreviated to as "HAp".

### BACKGROUND ART

For medical materials such as artificial bones, artificial joints, implant and the like, metals such as titanium, stainless steel, and the like, or ceramics are used. In particular, titanium has excellent corrosion resistance, high impact strength, is hard and tough, has good compatibility with living organisms and can be bonded to bone, and has elasticity close to that of bone. However, titanium cannot be utilized for artificial organs having a sliding part such as artificial joints since it is easily worn. Further, mold processing of titanium is more difficult than stainless steel. On the other hand, among ceramics, hydroxyapatite (HAp) has begun to be used for dental implant. This is because HAp is an inorganic component of bones and teeth and is an excellent biomaterial having extremely good affinity with bones. However, HAp sintered by hot pressing shows a compressive strength of 200 MPa or more, but bending strength and impact strength thereof are low, and it is easily broken so that it is difficultly utilized for uses that require strength.

In consideration of characteristics of HAp and characteristics of a metal substrate, there has been known a method in which hydroxyapatite is fixed on a metal substrate. Heretofore, as this kind of method, it has been disclosed a method for preparing a hydroxyapatite (HAp) coating on stainless steel (316L) by a wet method using a supersaturated simulated body fluid (simulated body fluid) (SBF) (for example, Patent Document 1 (see Claim 1, paragraph [0001] and paragraph [0025])).

This method comprises a) a step of roughening a surface of a metal substrate using acetone, ethanol and deionized water by a known method, and ultrasonically cleaning to obtain a clean metal substrate; b) a step of drying the clean metal substrate at a temperature in the range of 50 to 60°C for about 1 to 2 hours, immersing the clean metal substrate in an aqueous solution of a protein with a concentration in the range of 4 to 10wt% at a temperature in the range of 40 to 50°C and at a pH in the range of 6.5 to 7 for about 2 to 4 hours, and then, cleaning it with water 3 to 4 times repeatedly, and drying it at a temperature in the range of 20 to 30°C for about 1 to 2 hours to obtain a dry surface-treated metal substrate; c) a step of immersing the dry surface-treated metal substrate in a simulated body fluid [SBF(N)] for 1 to 2 days at a temperature of 35 to 37°C and at a pH in the range of 6.5 to 7 to obtain a nucleated hydroxyapatite-coated metal substrate, cleaning it with deionized water 3 to 4 times, and drying the clean hydroxyapatite nucleated metal at a temperature in the range of 20 to 30°C for about 1 to 2 hours to obtain a dried hydroxyapatite nucleated metal substrate; and d) a step of immersing the dried hydroxyapatite nucleated metal substrate in another simulated body fluid [SBF(O)] for a time in the range of 2 to 4 days at a temperature of 35 to 37°C and at a pH in the range of 6 to 6.5, cleaning it with deionized water 3 to 4 times, and drying it at a temperature in the range of 20 to 30°C for about 1 to 2 hours to obtain a desired protein-mediated calcium hydroxyapatite (HAp) coating on the hydroxyapatite nucleated metal substrate.

US 5,077,132 discloses a biocompatible composite material. Inter alia, a hydroxyapatite-coated Ti substrate is disclosed which is manufactured by coating Ti having a surface roughness of 3.4 µm with HAp and sintering at 900 °C for 5 min in air or sintering at 1050 °C for 20 min. The preferred thickness of the HAp layer is 20-100µm.

### PRIOR ART DOCUMENT

### PATENT DOCUMENT

Patent Document 1: JP 2009-67669A

### DISCLOSURE OF INVENTION

### PROBLEM TO BE SOLVED BY THE INVENTION

In the method shown in Patent Document 1, an HAp coating having a thickness of 20 µm to 280 µm can be obtained on the surface of a metal substrate, but this method is a wet method, so that the number of the steps for producing the HAp coating is high, and the HAp coating cannot be produced on the surface of the metal substrate unless it takes a long time of several days or more. In addition, there was a problem that the surface hardness of the resulting HAp coating was low.

An object of the present invention is to provide a method for fixing hydroxyapatite on a surface of a titanium-based metal substrate with a small number of steps and a relatively short time. In addition, another object of the present invention is to provide a hydroxyapatite-coated metal material on which a hydroxyapatite layer, which can be a scaffold for osteoblasts to proliferate, is coated with high hardness.

### MEANS FOR SOLVING PROBLEM

A first aspect of the present invention is a method for fixing hydroxyapatite to titanium-based metal substrate which comprises a step of a subjecting a surface of a titanium-based metal substrate comprising pure titanium or a titanium alloy to surface roughening treatment so that a surface roughness Ra becomes 1.6 µm or more and 4.0 µm or less, a step of adhering hydroxyapatite powder to entire surface or a part of the surface of the titanium-based metal substrate subjected to the surface roughening treatment at a ratio of 25 mg/cm² or more and 70 mg/cm² or less, and a step of retaining the titanium-based metal substrate, to which the hydroxyapatite powder has been adhered, at a sintering temperature of 800°C or nigner to 1150 °C or lower for 3 hours or longer and 11 hours or shorter when an atmosphere is air, argon gas or carbon dioxide gas air, or retaining it at a sintering temperature of 1,050°C or higher for 3 hours or longer and 11 hours or shorter when the atmosphere is nitrogen gas, or retaining it at a sintering temperature of 900°C or higher for 3 hours or longer and 11 hours or shorter when the atmosphere is oxygen gas, to sinter the hydroxyapatite powder on the surface of the metal substrate.

A second aspect of the present invention is an invention based on the first aspect, and is characterized in that the titanium-based metal substrate is a metal injection molded substrate.

A third aspect of the present invention is a hydroxyapatite-coated metal material which comprises a hydroxyapatite layer having a thickness of 10 µm or more and 510 µm or less, which can be a scaffold for osteoblasts to proliferate, on a surface of a titanium-based metal substrate, a fixed amount of the hydroxyapatite of 5 mg/cm² or more and 50 mg/cm² or less, a hardness of a surface of the hydroxyapatite layer regulated by JIS-K5600-5-4 of 6H or more, and a Vickers hardness regulated by JIS-2-2244 of 250 HV or more and 500 HV or less.

A fourth aspect of the present invention is an invention based on the third aspect, and is characterized in that the titanium-based metal substrate is a metal injection molded substrate.

### EFFECT OF THE INVENTION

In the method of the first aspect of the present invention, after the hydroxyapatite powder is adhered to the surface of a titanium-based metal substrate which has been subjected to a surface roughening treatment so that the surface roughness Ra becomes 1.6 µm or more and 4.0 um or less with a prescribed ratio, the titanium-based metal substrate, to which the hydroxyapatite powder has been adhered is retained under an atmosphere of air, nitrogen gas, argon gas, oxygen gas or carbon dioxide gas air at a prescribed temperature for 3 hours or longer and 11 hours or shorter, so that the hydroxyapatite powder is sintered and fixed on the surface of the titanium-based metal substrate. Therefore, hydroxyapatite can be fixed on the surface of the titanium-based metal substrate with a small number of steps and a relatively short time in this sintering method as compared with the wet method. At the fixed hydroxyapatite layer, it is possible to proliferate osteoblasts.

In the method of the second aspect of the present invention, the titanium-based metal substrate is a metal injection molded substrate, so that a hydroxyapatite-coated metal material having a complicated shape can be produced.

The hydroxyapatite-coated metal material of the third aspect of the present invention which is obtainable by the above-mentioned process has characteristics that it has a hydroxyapatite layer, which can be a scaffold of osteoblasts to proliferate, on the surface of a titanium-based metal substrate with a prescribed thickness and a prescribed fixed amount per a unit area, and a surface of the hydroxyapatite layer has a high hardness of 6H or more in a hardness of a pencil hardness test, and 250 HV or more and 500 HV or less in a Vickers hardness regulated in JIS-2-2244.

In the hydroxyapatite-coated metal material of the fourth aspect of the present invention, the titanium-based metal substrate is a metal injection molded substrate, so that it can be applied to a medical material having a complicated shape.

### BRIEF DESCRIPTION OF DRAWINGS

FIG. 1 is a process diagram of a method for fixing hydroxyapatite to titanium-based metal substrate according to an embodiment of the present invention;
FIG. 2 is a schematic view until a hydroxyapatite-coated metal material is produced according to an embodiment of the present invention;
FIG. 3 is a diagram showing a situation in which CaCO₃ is formed as raising the temperature, and CaTiO₃ is formed when the temperature reaches a prescribed temperature at the time of heating of the present embodiment;
FIG. 4 is a schematic view showing a situation in which hydroxyapatite (HAp) is bound to a TiO₂ layer at the time of heating of the present embodiment;
FIG. 5 is a schematic view showing a situation in which HAp and TiO₂ are bound with CaTiO₃ as a bonding point at the time of heating of the present embodiment; and
FIG. 6 is a diagram showing a heat pattern when hydroxyapatite powder of Example 1 is sintered on the surface of a titanium-based metal substrate.

### BEST MODE(S) FOR CARRYING OUT THE INVENTION

Next, an embodiment to carry out the present invention is explained with reference to the drawings.

### [Method for fixing hydroxyapatite to titanium-based metal substrate]

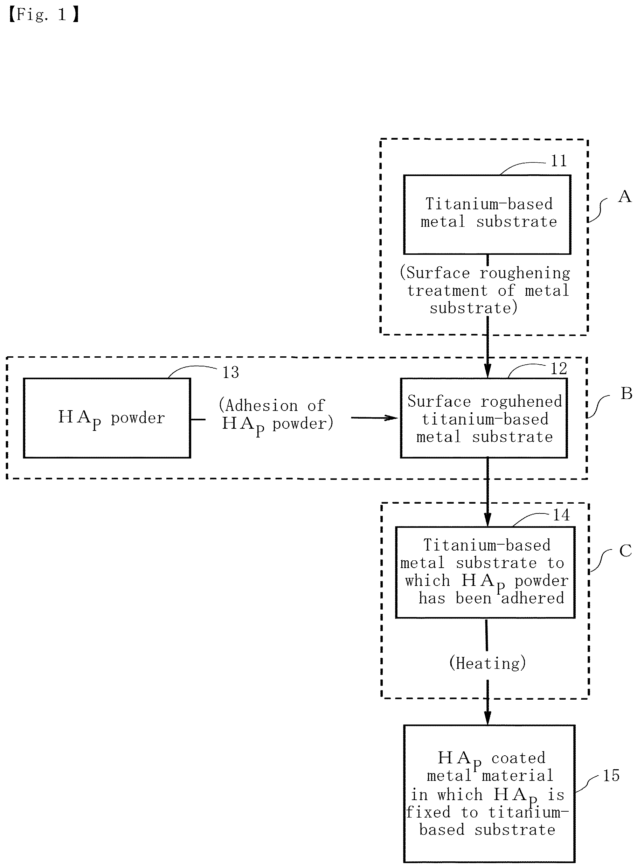

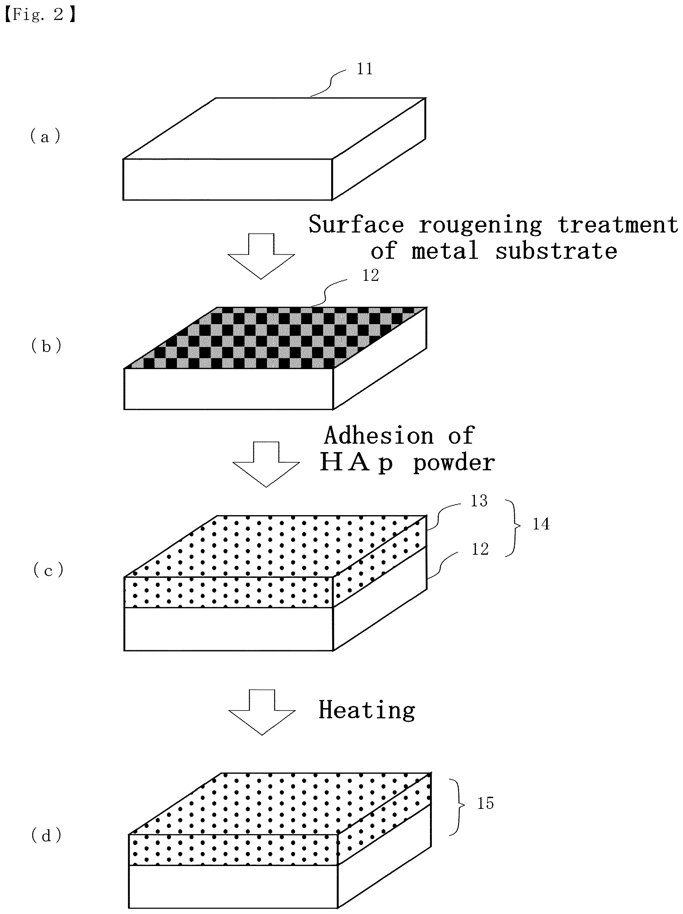

The method for fixing hydroxyapatite to titanium-based metal substrate of the embodiment of the present invention comprises, Step A of subjecting a surface of a titanium-based metal substrate 11 to a surface roughening treatment so that a surface roughness Ra thereof becomes 1.6 µm or more and 4.0 µm or less as shown in FIG. 1, FIG. 2(a) and FIG. 2(b), Step B of adhering hydroxyapatite powder 13 to entire surface or a part of the surface of the titanium-based metal substrate 12, which has been subjected to the surface roughening treatment as shown in FIG. 1 and FIG. 2(c), and Step C of retaining the titanium-based metal substrate 14, to which hydroxyapatite powder has been adhered, under a prescribed atmosphere at a temperature of 800°C or higher 1,150°C or lower for 3 hours or longer and 11 hours or shorter to sinter the hydroxyapatite powder to the surface of the metal substrate as shown in FIG. 1 and FIG. 2(c). According to this procedure, a hydroxyapatite-coated metal material 15 in which hydroxyapatite is fixed to the titanium-based metal substrate can be obtained.

### [Surface roughening treatment on surface of titanium-based metal substrate]

The metal substrate 11 used in the embodiment of the present invention is a pure titanium substrate or a titanium alloy substrate. As the pure titanium, titanium (for example, JIS type 2 titanium) regulated by JIS standard is mentioned, and as the titanium alloy, corrosion resistant titanium alloy (for example, JIS type 11, type 12 and type 13 titanium alloys), 64 titanium alloy (Ti6Al4V), etc., are mentioned. The shape of the metal substrate 11 can be determined from among plate-shaped, rod-shaped, columnar, tubular, etc., depending on the use such as artificial bones, artificial joints, implant, etc. When the metal substrate has a complicated shape, it is preferable to use a metal injection molded product. The surface roughening treatment of the surface of the titanium-based metal substrate is carried out by sandblasting treatment.

In the sandblasting treatment, alumina particles having an average particle diameter in the range of 30 µm to 300 µm are injected to the surface of the above-mentioned metal substrate at an injection pressure of, for example, 5.0 kg/cm². Thereafter, this metal substrate is immersed in a 10% caustic soda aqueous solution at 50°C for 2 minutes, further immersed in an 8% nitric acid aqueous solution at 20°C for 2 minutes, and then, the metal substrate is washed with pure water or ultrasonically cleaned in pure water. Alternatively, it is ultrasonically cleaned in pure water immediately after injection. By this sandblasting treatment, the surface roughness Ra of the titanium-based metal substrate is adjusted to 1.6 µm or more and 4.0 µm or less, and preferably 1.6 µm or more and 1.8 µm or less.

If the surface roughness Ra obtained by the sandblasting treatment is less than 1.6 um, when the hydroxyapatite powder is tried to be adhered as mentioned later, the degree of unevenness of the surface of the metal substrate is insufficient, and the powder difficultly adhered to the surface of the titanium-based metal substrate. Also, if it exceeds 4.0 um, the surface uneven shape of the molded product is too large so that uniform teeth or bones are difficultly formed. The above-mentioned surface roughness Ra is measured using a surface roughness measurement device (Surfest SJ-400 manufactured by Mitutoyo Corporation).

### [Hydroxyapatite powder]

The hydroxyapatite powder is spherical, and an average particle diameter thereof is preferably 20 µm or more and 40 µm or less. If it is less than 20 µm, adsorptivity to proteins and adhesive property to osteoblasts tend to be easily lowered, so that it is difficultly to form teeth or bones. If it exceeds 40 µm, the hydroxyapatite is difficultly fixed to the titanium-based metal substrate. The average particle diameter of the powder is measured by a laser diffraction method. As the hydroxyapatite powder, powders prepared by various synthetic methods can be used, and the crystallinity and the substitutability with various ions are not particularly limited. For example, hydroxyapatite in which the crystal structure site is partially replaced with a potassium ion, a sodium ion, a magnesium ion, a strontium ion, a fluoride ion, a carbonate ion, etc., may be used. As the commercially available hydroxyapatite powder, there may be mentioned a product number 289,396 available from Sigma-Aldrich Co.

### [Adhesive method of hydroxyapatite powder]

The hydroxyapatite powder 13 is uniformly adhered to entire surface or a part of the surface of the titanium-based metal substrate 12, which has been subjected to surface roughening treatment. The hydroxyapatite powder is adhered to the substrate 12 at a ratio of 25 mg/cm² or more and 50 mg/cm² or less. If it is less than 25 mg/cm², there is a fear of difficultly forming a hydroxyapatite layer with a sufficient thickness as a foothold (scaffold) for osteoblasts to proliferate, while if it exceeds 50 mg/cm², inconvenience of peeling is likely to occur. The adhered amount of the hydroxyapatite powder is further preferably 30 mg/cm² or more and 40 mg/cm² or less.

As a method of adhering the hydroxyapatite powder, there may be mentioned a method in which hydroxyapatite powder is applied by dropping from above the titanium-based metal substrate horizontally placed to coat thereon, then, the titanium-based metal substrate is turned over to remove excess powder by its own weight to form a hydroxyapatite powder layer having a uniform thickness on the titanium-based metal substrate, or a method in which the hydroxyapatite powder is stored in an opened container, and after contacting the titanium-based metal substrate with the hydroxyapatite powder in the container, the titanium-based metal substrate is pulled up to form a hydroxyapatite powder layer having a uniform thickness on the titanium-based metal substrate, and further a method in which the titanium-based metal substrate is buries in the hydroxyapatite powder in a container, then, the metal substrate is pulled up and the excessively adhered powder is removed to form a hydroxyapatite powder layer having a uniform thickness on the titanium-based metal substrate. According to these methods, the hydroxyapatite powder enters into the roughened recess portions of the titanium-based metal substrate and the hydroxyapatite powder adheres thereto.

### [Heating of titanium-based metal substrate to which HAp powder is adhered and sintering of HAp]

The titanium-based metal substrate 14 to which the hydroxyapatite powder is adhered is heated by an electric furnace, a microwave furnace, a gas furnace, etc. Heating is carried out under a prescribed atmosphere at a sintering temperature of 800°C or higher for 3 hours or longer and 11 hours or shorter. As the atmosphere, there may be mentioned an atmosphere of air, nitrogen gas, argon gas, oxygen gas or carbon dioxide gas absorptivity. Fixation of the hydroxyapatite to the titanium-based metal substrate differs depending on the kind of atmosphere. This is because decomposition of the hydroxyapatite differs depending on the kind of atmosphere. The atmosphere is most preferably carbon dioxide gas absorptivity atmosphere since can easily decompose the hydroxyapatite, and an atmosphere containing carbon dioxide gas absorptivity is more preferable. Sintering is carried out by retaining at a sintering temperature of 800°C or higher, and preferably at a sintering temperature of 1,150°C or lower for 3 hours or longer and 11 hours or shorter when the atmosphere is air, argon gas or carbon dioxide gas is air. Also, sintering is carried out by retaining at a sintering temperature of 1,050°C or higher, and preferably at a sintering temperature of 1,150°C or lower for 3 hours or longer and 11 hours or shorter when the atmosphere is nitrogen gas. Further, sintering is carried out by retaining at a sintering temperature of 900°C or higher, and preferably at a sintering temperature of 1,150°C or lower for 3 hours or longer and 11 hours or shorter when the atmosphere is oxygen gas. Also, the reason why the preferred upper limit of the sintering temperature is set to 1,150°C is that unnecessary heat energy consumption is to be suppressed. The rate of temperature rising from room temperature is preferably 6°C/min or more and 30°C/min or less for forming CaTiO₃ continuously from CaCO₃. The sintering time is preferably 6 hours or longer. In the titanium-based metal substrate, TiO₂ is formed on the surface of the substrate by oxygen in the atmosphere before sintering. After sintering, it is preferable to cool the furnace at a temperature lowering rate of 3°C/min or more and 10°C/min or less.

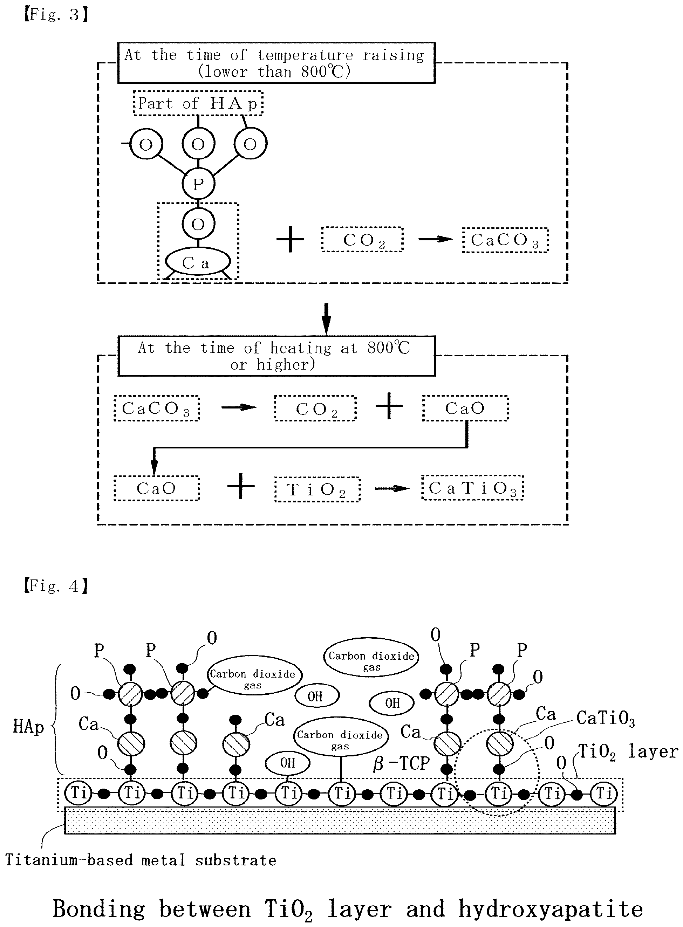

As shown in FIG. 3, when the sintering atmosphere is air or carbon dioxide (CO₂) gas and the atmosphere temperature is lower than 800°C, CaO which is a part of the hydroxyapatite and CO₂ are reacted to form CaCO₃. At this time, CO₂ in the atmosphere promotes decomposition of CaCO₃.

As shown in FIG. 3 and FIG. 4, when the atmosphere temperature becomes 800°C or higher, it reaches to the decomposition temperature of CaCO₃, and CaCO₃ is decomposed into CaO and CO₂ again. Reactive CaO formed after decomposition reacts with TiO₂ on the surface of the substrate to form CaTiO₃ with a perovskite structure on the surface of the titanium-based metal substrate and strong bonds with the surface of the substrate are formed.

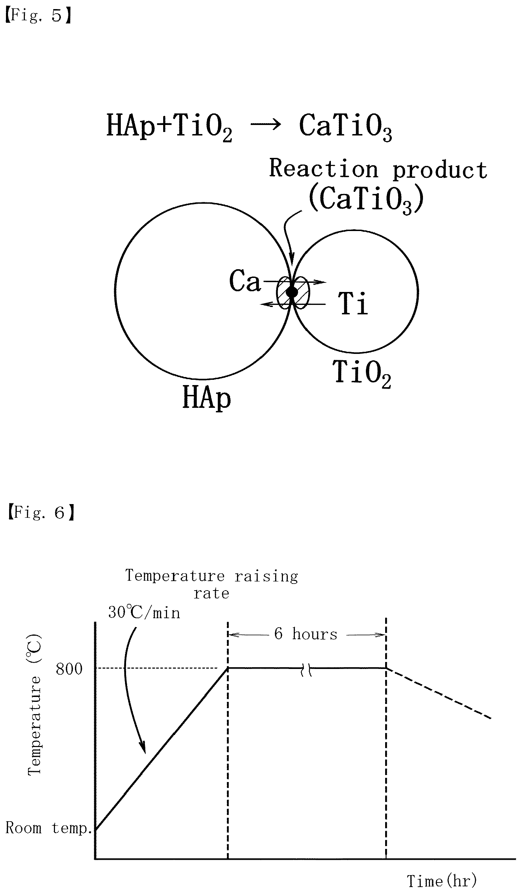

As shown in FIG. 5, CaTiO₃ which is the reaction product serves as bonding points between the hydroxyapatite (HAp) and TiO₂, whereby the hydroxyapatite layer is fixed to the titanium-based metal substrate with high hardness. When the atmosphere is air, argon gas or carbon dioxide gas, if the sintering temperature is lower than 800°C or the sintering time is shorter than 3 hours, the above-mentioned interaction is not sufficiently carried out and the hydroxyapatite powder is not sufficiently fixed to the substrate. When the atmosphere is nitrogen gas, if the sintering temperature is lower than 1,050°C or the sintering time is shorter than 3 hours, the above-mentioned interaction is not sufficiently carried out and the hydroxyapatite powder is not sufficiently fixed to the substrate. When the atmosphere is oxygen gas, if the sintering temperature is lower than 900°C or shorter than 3 hours, the above-mentioned interaction is not sufficiently carried out and the hydroxyapatite powder is not sufficiently fixed to the substrate.

### [Hydroxyapatite-coated metal material]

Returning to FIG. 1, FIG. 2(c) and FIG. 2(d), by heating the titanium-based metal substrate 14 on which HAp powder has coated under the above-mentioned conditions, a hydroxyapatite-coated metal material 15 to which the hydroxyapatite layer is coated with high hardness by sintering is produced. The thickness of the hydroxyapatite layer varies depending on the amount of the above-mentioned HAp powder 14 to be adhered, and is 10 µm or more and 510 µm or less. The fixed amount of the hydroxyapatite is 5 mg/cm² or more and 50 mg/cm² or less. If the thickness is less than 10 µm or the fixed amount is less than 5 mg/cm², there is a fear of difficultly forming a hydroxyapatite layer with a sufficient thickness as a foothold (scaffold) for osteoblasts to proliferate, while if the thickness exceeds 510 µm or the fixed amount exceeds 50 mg/cm², the hydroxyapatite layer after sintering is likely to peel off. The thickness of the hydroxyapatite layer is preferably 20 µm or more and 300 µm or less. Also, the fixed amount of the hydroxyapatite is preferably 5.5 mg/cm² or more and 40 mg/cm² or less. The hardness of the hydroxyapatite layer is 6H or more when it is measured by a pencil hardness test regulated in JIS-K5600-5-4. Further, the Vickers hardness regulated in JIS-2-2244 of the hydroxyapatite layer is 250 HV or more and 500 HV or less, preferably 400 HV or more

### EXAMPLES

Hereinafter, Examples of the present invention will be explained in detail with Comparative Examples.

### <Example 1>

As shown in FIG. 2(a), a pure titanium plate material having a thickness of 4 mm manufactured by CASTEM Co., Ltd., was cut into 10 mm in length and 10 mm in width to obtain a titanium substrate 11. The surface of this titanium substrate 11 was subjected to sandblasting treatment by injecting alumina particles having an average particle diameter of 280 µm at an injection pressure of 5.0 kg/cm². Thereafter, the titanium substrate was immersed in a 10% caustic soda aqueous solution at 50°C for 2 minutes, further immersed in an 8% nitric acid aqueous solution at 20°C for 2 minutes, and then, the titanium substrate was ultrasonically cleaned in pure water. According to this procedure, a titanium substrate 12 subjected to surface roughening treatment was obtained as shown in FIG. 2(b) by subjecting to surface roughening treatment. The surface roughness Ra of this titanium substrate 12 was about 1.7 um. On the other hand, commercially available hydroxyapatite powder (product number: 289396 manufactured by Sigma-Aldrich Co.) having an average particle diameter of 20 µm was prepared.

After the titanium substrate 12 subjected to surface roughening treatment was arranged horizontally, as shown in FIG. 2(c), hydroxyapatite powder was applied to the entire upper surface of titanium substrate 12 from above the titanium substrate 12 to have a uniform thickness, and adhered. The coated amount of the applied powder 13, that is, the adhered amount, was 25 mg/cm². Subsequently, the titanium substrate 14 to which the hydroxyapatite powder was adhered was placed in an electric furnace, and as shown in FIG. 5, the temperature was raised to 800°C at a rate of 30°C/min in an atmosphere and retained for 6 hours to sinter the hydroxyapatite powder. According to this procedure, as shown in FIG. 2(d), a hydroxyapatite-coated titanium material 15 in which a hydroxyapatite layer was fixed on the titanium substrate 12 subjected to surface roughening treatment was obtained.

The material of the titanium substrate which is a starting material of this hydroxyapatite-coated titanium material, the surface roughness after surface roughening of the titanium substrate, the average particle diameter of the HAp powder, the adhered amount of the HAp powder on the titanium substrate, and its sintering conditions (atmosphere, sintering temperature and sintering time) are shown in the following Table 1.

**[Table 1]**

| | Producing conditions of HAp coated titanium substrate | | | | | | | Evaluation of HAp coated titanium substrate | | | | | | | |
|---|---|---|---|---|---|---|---|---|---|---|---|---|---|---|---|
| | Titanium substrate | | HAp powder | | Sintering conditions | | | Fixed amount of HAp (mg/cm²) | Thickness of HAp layer (µm) | Presence or absence of Ca, P by XRF | Presence or absence of HAp component by XRD | Pencil hardness | Vickers hardness (HV) | Presence or absence of peeling by impact | Osteoblasts |
| | Material | Surface roughness (µm) | Average particle diameter (µm) | Attached amount on substrate (mg/cm²) | Atmosphere | Temperature (°C) | Time (hr) | | | | | | | | |
| Example 1 | Pure titanium | 1.7 | 20 | 25 | Air | 800 | 6.0 | 5.7 | 14 | Present | Present | ≧6H | 259 | None | Possible |
| Example 2 | Pure titanium | 1.7 | 30 | 35 | Air | 900 | 6.0 | 11.7 | 58 | Present | Present | ≧6H | 321 | None | Possible |
| Example 3 | Pure titanium | 1.6 | 30 | 40 | Air | 1,000 | 6.0 | 14.9 | 503 | Present | Present | ≧6H | 387 | None | Possible |
| Example 4 | Pure titanium | 1.7 | 40 | 40 | Air | 1, 150 | 6.0 | 15.2 | 510 | Present | Present | ≧6H | 526 | None | Possible |
| Example 5 | Pure titanium | 1.7 | 30 | 60 | Air | 1,100 | 3.0 | 29.0 | 398 | Present | Present | ≧6H | 265 | None | Possible |
| Example 6 | Pure titanium | 1.7 | 20 | 70 | Air | 1,100 | 5.0 | 49.3 | 471 | Present | Present | ≧6H | 438 | None | Possible |
| Example 7 | Pure titanium | 1.7 | 30 | 65 | Air | 1,100 | 9.0 | 39.2 | 258 | Present | Present | ≧6H | 257 | None | Possible |
| Example 8 | Pure titanium | 1.7 | 30 | 70 | Air | 1,100 | 11.0 | 46.2 | 358 | Present | Present | ≧6H | 374 | None | Possible |
| Comparative Example 1 | Pure titanium | 1.5 | 30 | 25 | Air | 800 | 6.0 | 1.6 | Measurement impossible | Present | None | - | - | - | Impossible |
| Comparative Example 2 | Pure titanium | 1.7 | 30 | 25 | Air | 750 | 6.0 | 4.8 | Measurement impossible | None | None | - | - | - | Impossible |
| Comparative Example 3 | Pure titanium | 1.7 | 30 | 70 | Air | 1,200 | 6.0 | 50.4 | 770 | Present | Present | ≧6H | 445 | Present | Possible |
| Comparative Example 4 | Pure titanium | 1.7 | 30 | 75 | Air | 1,100 | 6.0 | 54.8 | 625 | Present | Present | ≧6H | 519 | Present | Possible |
| Comparative Example 5 | Pure titanium | 1.7 | 30 | 60 | Air | 1,100 | 2.0 | 27.5 | 239 | Present | Present | ≧6H | 567 | Present | Possible |
| Comparative Example 6 | Pure titanium | 1.7 | 30 | 70 | Air | 1,100 | 12.0 | 65.3 | 780 | Present | Present | ≧6H | 642 | Present | Possible |

### <Examples 2 to 8 and Comparative Examples 1 to 6>

With regard to the case where the atmosphere is air, the material of the titanium substrate which is a starting material, the surface roughness of the titanium substrate after surface roughening, the average particle diameter of the HAp powder, the adhered amount of the HAp powder on the titanium substrate, and the sintering conditions thereof (atmosphere, sintering temperature and sintering time) were each changed as shown in the above-mentioned Table 1, and hydroxyapatite-coated titanium materials of Examples 2 to 8 and Comparative Examples 1 to 6 were obtained in the same manner as in Example 1 except for the above.

### <Examples 9 and 10 and Comparative Example 7>

With regard to the case where the atmosphere is nitrogen gas, the material of the titanium substrate which is a starting material, the surface roughness of the titanium substrate after surface roughening, the average particle diameter of the HAp powder, the adhered amount of the HAp powder on the titanium substrate, and the sintering conditions thereof (atmosphere, sintering temperature and sintering time) were each changed as shown in the following Table 2, and hydroxyapatite-coated titanium materials of Examples 9 and 10 and Comparative Example 7 were obtained in the same manner as in Example 1 except for the above.

**[Table 2]**

| | Producing conditions of HAp coated titanium substrate | | | | | | | Evaluation of HAp coated titanium substrate | | | | | | | |
|---|---|---|---|---|---|---|---|---|---|---|---|---|---|---|---|
| | Titanium substrate | | HAp powder | | Sintering conditions | | | Fixed amount of HAp (mg/cm²) | Thickness of HAp layer (µm) | Presence or absence of Ca, P by XRF | Presence or absence of HAp component by XRD | Pencil hardness | Vickers hardness (HV) | Presence or absence of peeling by impact | Osteoblasts |
| | Material | Surface roughness (µm) | Average particle diameter (µm) | Attached amount on substrate (mg/cm²) | Atmosphere | Temperature (°C) | Time (hr) | | | | | | | | |
| Example 9 | Pure titanium | 1.7 | 30 | 25 | N₂ | 1,050 | 6.0 | 5.5 | 35 | Present | Present | ≧6H | 436 | None | Possible |
| Example 10 | Pure titanium | 1.7 | 40 | 25 | N₂ | 1,150 | 6.0 | 7.5 | 161 | Present | Present | ≧6H | 451 | None | Possible |
| Comparative Example 7 | Pure titanium | 1.7 | 40 | 25 | N₂ | 1,000 | 6.0 | 3.4 | Measurement impossible | None | None | - | - | - | Impossible |
| Example 11 | Pure titanium | 1.7 | 30 | 25 | Ar | 850 | 6.0 | 8.9 | 39 | Present | Present | ≧6H | 612 | None | Possible |
| Example 12 | Pure titanium | 1.7 | 30 | 35 | Ar | 900 | 6.0 | 12.7 | 81 | Present | Present | ≧6H | 664 | None | Possible |
| Example 13 | Pure titanium | 1.7 | 30 | 25 | Ar | 950 | 6.0 | 8.7 | 143 | Present | Present | ≧6H | 567 | None | Possible |
| Example 14 | Pure titanium | 1.8 | 30 | 25 | Ar | 1,000 | 6.0 | 7.6 | 295 | Present | Present | ≧6H | 462 | None | Possible |
| Example 15 | Pure titanium | 1.7 | 30 | 25 | O₂ | 900 | 6.0 | 8.3 | 85 | Present | Present | ≧6H | 663 | None | Possible |
| Example 16 | Pure titanium | 1.7 | 30 | 30 | O₂ | 950 | 6.0 | 10.0 | 150 | Present | Present | ≧6H | 635 | None | Possible |
| Example 17 | Pure titanium | 1.7 | 30 | 25 | O₂ | 1,000 | 6.0 | 7.9 | 355 | Present | Present | ≧6H | 736 | None | Possible |
| Comparative Example 8 | Pure titanium | 1.7 | 30 | 25 | O₂ | 850 | 6.0 | 4.7 | Measurement impossible | None | None | - | - | - | Impossible |
| Example 18 | Pure titanium | 1.7 | 30 | 25 | CO₂ | 850 | 6.0 | 8.9 | 27 | Present | Present | ≧6H | 526 | None | Possible |
| Example 19 | Pure titanium | 1.7 | 30 | 30 | CO₂ | 900 | 6.0 | 12.8 | 68 | Present | Present | ≧6H | 486 | None | Possible |
| Example 20 | Pure titanium | 1.7 | 30 | 55 | CO₂ | 950 | 6.0 | 27.5 | 141 | Present | Present | ≧6H | 537 | None | Possible |
| Example 21 | Pure titanium | 1.7 | 30 | 60 | CO₂ | 1,000 | 6.0 | 42.7 | 202 | Present | Present | ≧6H | 472 | None | Possible |

### <Examples 11 to 14>

With regard to the case where the atmosphere is argon gas, the material of the titanium substrate which is a starting material, the surface roughness of the titanium substrate after surface roughening, the average particle diameter of the HAp powder, the adhered amount of the HAp powder on the titanium substrate, and the sintering conditions thereof (atmosphere, sintering temperature and sintering time) were each changed as shown in the above-mentioned Table 2, and hydroxyapatite-coated titanium materials of Examples 11 to 14 were obtained in the same manner as in Example 1 except for the above.

### <Examples 15 to 17 and Comparative Example 8>

With regard to the case where the atmosphere is oxygen gas, the material of the titanium substrate which is a starting material, the surface roughness of the titanium substrate after surface roughening, the average particle diameter of the HAp powder, the adhered amount of the HAp powder on the titanium substrate, and the sintering conditions thereof (atmosphere, sintering temperature and sintering time) were each changed as shown in the above-mentioned Table 2, and hydroxyapatite-coated titanium materials of Examples 15 to 17 and Comparative Example 8 were obtained in the same manner as in Example 1 except for the above.

### <Examples 18 to 21>

With regard to the case where the atmosphere is carbon dioxide gas, the material of the titanium substrate which is a starting material, the surface roughness of the titanium substrate after surface roughening, the average particle diameter of the HAp powder, the adhered amount of the HAp powder on the titanium substrate, and the sintering conditions thereof (atmosphere, sintering temperature and sintering time) were each changed as shown in the above-mentioned Table 2, and hydroxyapatite-coated titanium materials of Examples 18 to 21 were obtained in the same manner as in Example 1 except for the above.

### <Comparative evaluation test>

With regard to samples of 29 kinds of the hydroxyapatite-coated titanium materials obtained in Examples 1 to 21 and Comparative Examples 1 to 8, the surface properties were evaluated by the following items. The results of these evaluations are shown in the above-mentioned Table 1 and Table 2. In addition, with regard to the sample of the hydroxyapatite-coated titanium material of Example 20, the adhesion rate of osteoblasts was examined.

### (1) Fixed amount of HAp

The fixed amount of the hydroxyapatite is measured by placing a prescribed amount of hydroxyapatite on a titanium substrate whose mass has been measured before sintering, and after sintering, the hydroxyapatite that has not been fixed was removed from the substrate with a soft brush, and the mass thereof was measured and the mass of titanium before sintering was subtracted to make a fixed amount of the hydroxyapatite.

### (2) Thickness of HAp layer

The thicknesses of the substrate before sintering and after sintering were measured by a micrometer (straight-ahead type blade micrometer BLM-50MX manufactured by Mitutoyo Corporation) and obtained.

### (3) Presence or absence of peaks of Ca and P by fluorescent X-ray analysis

Elements at the surface of each sample was analyzed using a fluorescent X-ray analyzer (X-ray Fluorescence: XRF) (Model Name: JSX-3222 manufactured by JEOL Ltd.). It was measured by making the radiation source element Rh, the tube voltage 30.0 kV, the tube current 0.150 mA, and the live time 1,200 seconds. When both peak waveforms corresponding to Ca and P, which are inorganic components of HAp, were observed in this XRF analysis, it was defined as "HAp component present", and when both peak waveforms were not observed, it was defined as "no HAp component".

### (4) Presence or absence of peak of HAp component by X-ray diffraction

The substances at the surface of each sample were analyzed using an X-ray diffraction (X-ray diffraction: XRD) device (Model Name: RINT2000 manufactured by Rigaku Corporation). The measurement range was 5° to 60°, the scanning speed was 4 deg/min, the voltage was 40 kV, the current was 20 mA, the slit width was 1-1-0.15°, and a CuKa line (characteristic X-ray) and a monochromator were used. When both peak waveforms corresponding to P and Ca, which are inorganic components of HAp, were observed in this XRD measurement, it was defined as "HAp component present", and when both peak waveforms were not observed, it was defined as "no HAp component".

### (5) Abrasion strength (pencil hardness) of fixed HAp layer

A pencil hardness test was carried out to measure the wear strength of the fixed HAp layer. Specifically, a pencil hardness tester (Allgood 054 series) was used and a peeling test of each sample fixed on the surface of the titanium substrate was carried out. Only the wood portion of the Mitsubishi uni pencil for the pencil hardness test was scraped to expose only the core by 5 mm to 6 mm. This pencil was set to the tester, and retained in the state that a load of 750 g ± 10 g is applied to the tip of the lead of the pencil with an angle of 45° ± 1° and pushed with a speed of 0.5 mm/sec to 1 mm/sec at a distance of at least 7 mm. The powder of the lead of the pencil adhered to the surface of the sample was wiped off with a soft cloth, and scratches or indentations were confirmed. The pencil hardness softer just before the scratched pencil was made the pencil hardness of the HAp layer. This test corresponds to the JIS standard of JIS-K5600-5-4.

### (6) Adhesion strength (Vickers hardness) of fixed HAp layer

A Vickers hardness test was carried out to measure the adhesion strength of the fixed HAp layer. The Vickers hardness is a Vickers hardness regulated by JIS 22244, and, for example, pressure was applied to five different points on the surface of the sample, the average value of resistance to plastic deformation thereby was calculated, and this average value thereof was made the Vickers hardness.

### (7) Adhesion strength (impact strength) of fixed HAp layer

A free fall test was carried out by the following method to measure the adhesion strength of the fixed HAp layer. A drop height was made 80 cm (free fall energy: 5.3J), the hydroxyapatite-coated titanium material to which the hydroxyapatite layer has fixed was freely fallen five times on the surface of a smooth concrete floor material, and whether the hydroxyapatite layer on the surface of the sample is peeled off or not was evaluated. This impact test is an impact test in which, when the sample is made a glass plate, with such a degree that the glass plate is broken by free fall from a height of 20 cm.

### (8) SEM observation of fixed HAp layer

The surface properties of the fixed HAp layer were observed using a scanning type electron microscope (Scanning Electron Microscope: SEM) (HITACHI S-4800). It was investigated whether or not there was a gap in the fixed HAp particles, and if it presents, whether or not osteoblasts could invade and fix to this gap and can be a foothold (scaffold) for proliferation.

### (9) Adhesion rate of osteoblasts to fixed HAp layer

Whether or not osteoblasts could proliferate on the fixed HAp layer was examined by the adhesion rate of osteoblasts to the HAp layer. This adhesion rate was obtained by the following method.

A titanium substrate or a titanium substrate fixed with a hydroxyapatite layer was fixed on the edge of a 35 mm culture dish with a transparent adhesive sheet. MC3T3-El cells were seeded with 1.5 × 10⁵ cells and cultured at 37°C for 24 hours. After cultivation, the cells were observed using an optical microscope, and the adhesion rate of osteoblasts was calculated by the following equation using, as a control, the portion other than the titanium substrate was fixed or the titanium substrate, to which a hydroxyapatite layer had been fixed was fixed. The number of the samples was made 3 and the average value thereof was obtained. Osteoblasts adhesion rate (%) = number of cells on sample/number of cells on untreated substrate (control)

As clearly seen from Table 1, in the sample of Comparative Example 1 in which sintering was carried out in air, the surface roughness Ra of the titanium substrate, which has been subjected to surface roughening treatment, was 1.5 µm so that surface roughening was not sufficiently carried out, and the fixed amount of HAp on the titanium substrate was 1.6 mg/cm², which was too small. Therefore, whereas both peak waveforms corresponding to Ca and P which were considered to be derived from HAp by fluorescent X-rays were observed, the thickness of HAp could not be measured, and the peak waveform of the HAp component by XRD was not observed. The evaluations of the pencil hardness test, Vickers hardness test and impact test (hereinafter referred to as pencil hardness test, etc.) were not carried out. As a result of SEM observation of osteoblasts, invasion and fixation of osteoblasts were impossible.

In the sample of Comparative Example 2 in which sintering was carried out in air, since the sintering temperature was 750°C, the fixed amount of HAp on the titanium substrate was 4.8 mg/cm², which was too small. Therefore, neither the peak waveform corresponding to Ca and P, which is considered to be derived from HAp by fluorescent X-rays, was observed, and the thickness of HAp could not be measured, further the peak waveform of the HAp component by XRD was not observed. No evaluation such as pencil hardness test, etc., was carried out. As a result of SEM observation of osteoblasts, invasion and fixation of osteoblasts were impossible.

In the sample of Comparative Example 3 in which sintering was carried out in air, the adhered amount of the HAp powder on the titanium substrate was too much so that the fixed amount of HAp on the titanium substrate was 50.4 mg/cm², which was too much, and the thickness of HAp was 770 um, which was too thick. Therefore, the HAp layer was peeled off from the surface of the titanium substrate in the impact test. The pencil hardness was 6H or more and the Vickers hardness was 445 HV, and as a result of SEM observation of osteoblasts, invasion and fixation of osteoblasts were possible.

In the sample of Comparative Example 4 in which sintering was carried out in air, the adhered amount of the HAp powder was 75 mg/cm², which was too much, so that the fixed amount of HAp on the titanium substrate was 54.4 mg/cm², which was too much, and the thickness of HAp was 625 um, which was too thick. The HAp layer was peeled off from the surface of the titanium substrate in the impact test. The pencil hardness was 6H or more and the Vickers hardness was 519 HV, and as a result of SEM observation of osteoblasts, invasion and fixation of osteoblasts were possible.

In the sample of Comparative Example 5 in which sintering was carried out in air, the sintering time was 2.0 hours, which was too short, the fixed amount of HAp on the titanium substrate was 27.5 mg/cm² and the thickness of HAp was 239 um, but the HAp layer was peeled off from the surface of the titanium substrate in the impact test. It is considered that this is because fixation of HAp on the titanium substrate could not be carried out. The pencil hardness was 6H or more and the Vickers hardness was 567 HV, and as a result of SEM observation of osteoblasts, invasion and fixation of osteoblasts were possible.

In the sample of Comparative Example 6 in which sintering was carried out in air, the sintering time was 12.0 hours, which was too long, the fixed amount of HAp on the titanium substrate was 65.3 mg/cm², which was too much and the thickness of HAp was 780 um, which was too thick. Therefore, the HAp layer was peeled off from the surface of the titanium substrate in the impact test. The pencil hardness was 6H or more and the Vickers hardness was 642 HV, and as a result of SEM observation of osteoblasts, invasion and fixation of osteoblasts were possible.

As clearly seen from Table 2, in the sample of Comparative Example 7 in which sintering was carried out in nitrogen gas atmosphere, the sintering temperature was 1,000°C so that the fixed amount of HAp on the titanium substrate was 3.4 mg/cm², which was too little. Therefore, neither the peak waveform corresponding to Ca and P, which is considered to be derived from HAp by fluorescent X-rays, was observed, and the thickness of HAp could not be measured, further the peak waveform of the HAp component by XRD was not observed. Evaluations such as the pencil hardness test, etc., were not carried out. As a result of SEM observation of osteoblasts, invasion and fixation of osteoblasts were impossible.

In the sample of Comparative Example 8 in which sintering was carried out in oxygen gas atmosphere, the sintering temperature was 850°C so that the fixed amount of HAp on the titanium substrate was 4.7 mg/cm², which was too little. Therefore, neither the peak waveform corresponding to Ca and P, which is considered to be derived from HAp by fluorescent X-rays, was observed, and the thickness of HAp could not be measured, further the peak waveform of the HAp component by XRD was not observed. Evaluations such as the pencil hardness test, etc., were not carried out. As a result of SEM observation of osteoblasts, invasion and fixation of osteoblasts were impossible.

To the contrary, in the samples of Examples 1 to 8 in which sintering was carried out in air, Examples 9 and 10 in which sintering was carried out in nitrogen gas atmosphere, 11 to 14 in which sintering was carried out in argon gas atmosphere, Examples 15 to 17 in which sintering was carried out in oxygen gas atmosphere, and Examples 18 to 21 in which sintering was carried out in carbon dioxide gas atmosphere, the peaks of Ca and P elements in the HAp composition were confirmed from fluorescent X-ray analysis in all the samples. Also, the peak of crystal structure of HAp was confirmed from the XRD (X-ray diffraction) analysis. Further, in the adhesion between the titanium substrate and HAp, it was confirmed that a part of the crystal structure of HAp was changed to tricalcium phosphate (β-TCP) and CaTiO₃ with a perovskite structure was formed by the interaction of TiO₂ and HAp formed on the surface of the titanium substrate from analyses of XRD peak analysis and XRF (fluorescent X-rays). According to this, it was inferred that the HAp layer was strongly bonded to the titanium substrate. That is, it was inferred that HAp was fixed to the surface of the titanium substrate.

In addition, in the samples of Examples 1-3, 5-10, 14, 19 and 21 the HAp layer showed the hardness that these had a hardness of 6H or more as a result of the pencil hardness test and the Vickers hardness was in the range of 250 HV or more and 500 HV or less. Moreover, in the impact strength by the free fall test, all of the HAp layers were not peeled off and had sufficient strength. In particular, it was confirmed that, in the carbon dioxide gas, the thickness could be increased while maintaining the adhesiveness of the hydroxyapatite layer to the substrate and hardness than the other gases.

Further, in the samples of Examples 1 to 21, a gap was confirmed between HAp particles constituting the fixed HAp layer from SEM observation, and a foothold (scaffold) in which osteoblasts proliferate is formed whereby invasion and fixation of osteoblasts were confirmed to be possible. In the sample of Example 20, osteoblasts adhered to the fixed HAp layer at a high rate of 84.3%. Samples of Examples 4, 11-13, 15-17, 18 and 20 do not fall under the scope of claim 3.

### INDUSTRIAL APPLICABILITY

The hydroxyapatite-coated metal material of the present invention serves as a foothold (scaffold) for osteoblasts to proliferate, and can be utilized for medical materials such as artificial bones, artificial joints, implant, etc.

## Claims

1. A method for fixing hydroxyapatite to a titanium-based metal substrate which comprises a step of subjecting a surface of a titanium-based metal substrate comprising pure titanium or a titanium alloy to surface roughening treatment so that a surface roughness Ra becomes 1.6 µm or more and 4.0 µm or less,
a step of adhering hydroxyapatite powder to entire surface or a part of the surface of the titanium-based metal substrate, which has been subjected to the surface roughening treatment, at a ratio of 25 mg/cm² or more and 70 mg/cm² or less, and
a step of retaining the titanium-based metal substrate, to which the hydroxyapatite powder has been adhered,
at a sintering temperature of 800°C or higher to 1,150 °C or lower for 3 hours or longer and 11 hours or shorter when an atmosphere is air, argon gas or carbon dioxide gas, or
retaining the titanium-based metal substrate at a sintering temperature of 1,050°C or higher for 3 hours or longer and 11 hours or shorter when the atmosphere is nitrogen gas, or
retaining the titanium-based metal substrate at a sintering temperature of 900°C or higher for 3 hours or longer and 11 hours or shorter when the atmosphere is oxygen gas,
to sinter the hydroxyapatite powder on the surface of the metal substrate.

2. The method according to Claim 1, wherein the titanium-based metal substrate is a metal injection molding substrate.

3. A hydroxyapatite-coated metal material which comprises a hydroxyapatite layer having a thickness of 10 µm or more and 510 µm or less, which can be a scaffold for osteoblasts to proliferate, on a surface of a titanium-based metal substrate, a fixed amount of the hydroxyapatite of 5 mg/cm² or more and 50 mg/cm² or less, a hardness on the surface of the hydroxyapatite layer regulated by JIS-K5600-5-4 of 6H or more, and a Vickers hardness regulated by JIS-2-2244 of 250 HV or more and 500 HV or less.

4. The hydroxyapatite-coated metal material according to Claim 3, wherein the titanium-based metal substrate is a metal injection molding substrate.

## Patentansprüche

1. Verfahren zur Fixierung von Hydroxyapatit auf einem Metallsubstrat auf Titanbasis, das einen Schritt umfasst, bei dem eine Oberfläche eines Metallsubstrats auf Titanbasis, das reines Titan oder eine Titanlegierung umfasst, einer Oberflächenaufrauhungsbehandlung unterzogen wird, so dass eine Oberflächenrauheit Ra 1,6 µm oder mehr und 4,0 µm oder weniger wird,
einen Schritt des Anhaftens von Hydroxyapatitpulver auf der gesamten Oberfläche oder einem Teil der Oberfläche des Metallsubstrats auf Titanbasis, das der Oberflächenaufrauhungsbehandlung unterzogen wurde, in einem Verhältnis von 25 mg/cm² oder mehr und 70 mg/cm² oder weniger, und
einen Schritt des Haltens des Metallsubstrats auf Titanbasis , an dem das Hydroxyapatit-Pulver haften geblieben ist,
bei einer Sintertemperatur von 800 °C oder höher bis 1.150 °C oder niedriger für 3 Stunden oder länger und 11 Stunden oder kürzer, wenn die Atmosphäre Luft, Argongas oder Kohlendioxidgas ist, oder
Halten des Metallsubstrats auf Titanbasis bei einer Sintertemperatur von 1.050°C oder höher für 3 Stunden oder länger und 11 Stunden oder kürzer, wenn die Atmosphäre Stickstoffgas ist, oder
Halten des Metallsubstrats auf Titanbasis bei einer Sintertemperatur von 900°C oder höher für 3 Stunden oder länger und 11 Stunden oder kürzer, wenn die Atmosphäre Sauerstoffgas ist,
um das Hydroxyapatitpulver auf der Oberfläche des Metallsubstrats zu sintern.

2. Verfahren nach Anspruch 1, wobei das Metallsubstrat auf Titanbasis ein Metallspritzgusssubstrat ist.

3. Ein mit Hydroxyapatit beschichtetes Metallmaterial, das eine Hydroxyapatitschicht mit einer Dicke von 10 µm oder mehr und 510 µm oder weniger, die ein Gerüst für die Proliferation von Osteoblasten sein kann, auf einer Oberfläche eines Metallsubstrats auf Titanbasis, eine feste Menge des Hydroxyapatits von 5 mg/cm² oder mehr und 50 mg/cm² oder weniger, eine durch JIS-K5600-5-4 geregelte Härte auf der Oberfläche der Hydroxyapatitschicht von 6H oder mehr und eine durch JIS-2-2244 geregelte Vickershärte von 250 HV oder mehr und 500 HV oder weniger umfasst.

4. Hydroxyapatit-beschichtetes Metallmaterial nach Anspruch 3, wobei das Metallsubstrat auf Titanbasis ein Metall-Spritzgusssubstrat ist.

## Revendications

1. Procédé de fixation d'hydroxyapatite sur un substrat métallique à base de titane qui comprend une étape consistant à soumettre une surface d'un substrat métallique à base de titane comprenant du titane pur ou un alliage de titane à un traitement de rugosité de surface de sorte qu'une rugosité de surface Ra devienne supérieure ou égale à 1,6 µm et inférieure ou égale à 4,0 um,
une étape consistant à faire adhérer de la poudre d'hydroxyapatite à la surface entière ou à une partie de la surface du substrat métallique à base de titane, qui a été soumis au traitement de rugosité de surface, à un taux de 25 mg/cm² ou plus et de 70 mg/cm² ou moins, et
une étape consistant à maintenir le substrat métallique à base de titane , auquel la poudre d'hydroxyapatite a été collée,
à une température de frittage de 800 °C ou plus à 1150 °C ou moins pendant 3 heures ou plus et 11 heures ou moins lorsque l'atmosphère est de l'air, de l'argon ou du dioxyde de carbone, ou
maintenir le substrat métallique à base de titane à une température de frittage de 1050 °C ou plus pendant 3 heures ou plus et 11 heures ou moins lorsque l'atmosphère est de l'azote gazeux, ou
maintenir le substrat métallique à base de titane à une température de frittage de 900°C ou plus pendant 3 heures ou plus et 11 heures ou moins lorsque l'atmosphère est constituée d'oxygène gazeux,
pour fritter la poudre d'hydroxyapatite sur la surface du substrat métallique.

2. Méthode selon la revendication 1, dans laquelle le substrat métallique à base de titane est un substrat de moulage par injection de métal.

3. Matériau métallique recouvert d'hydroxyapatite qui comprend une couche d'hydroxyapatite d'une épaisseur de 10 µm ou plus et de 510 um ou moins, qui peut être un échafaudage pour la prolifération des ostéoblastes, sur une surface d'un substrat métallique à base de titane, une quantité fixe d'hydroxyapatite de 5 mg/cm² ou plus et de 50 mg/cm² ou moins, une dureté à la surface de la couche d'hydroxyapatite réglementée par la norme JIS-K5600-5-4 de 6H ou plus, et une dureté Vickers réglementée par la norme JIS-2-2244 de 250 HV ou plus et de 500 HV ou moins.

4. Matériau métallique revêtu d'hydroxyapatite selon la revendication 3, dans lequel le substrat métallique à base de titane est un substrat de moulage par injection de métal.
